# EUROPEAN PATENT SPECIFICATION

(11) **EP 2 114 004 B1**
(45) Date of publication and mention of the grant of the patent: **17.12.2014**
(21) Application number: 07828888.3
(22) Date of filing: 25.09.2007
(51) Int. Cl.: A61B 8/12, H02N 2/16

(54) **ULTRASONIC OPERATION DEVICE AND MICROTUBE INSIDE SYSTEM**
ULTRASCHALL-BETRIEBSEINRICHTUNG UND MIKRORÖHREN-INSIDE-SYSTEM
DISPOSITIF DE FONCTIONNEMENT ULTRASONIQUE ET SYSTÈME INTERNE À MICROTUBE

(30) Priority: 25.09.2006 JP 2006259788
(43) Date of publication of application: 04.11.2009
(73) Proprietor: National University Corporation Tokyo University of Agriculture and Technology, Fuchu-shi, Tokyo 183-8538 (JP)
(72) Inventor: MASHIMO, Tomoaki, Fuchu-shi Tokyo 183-8538 (JP); TOYAMA, Shigeki, Fuchu-shi Tokyo 183-8538 (JP)
(74) Representative: Isarpatent
(86) International application number: PCT/JP2007/069147
(87) International publication number: WO 2008/038817

(56) References cited:
- DE-A1- 4 435 996
- JP-A- 02 070 275
- JP-A- 03 198 672
- JP-A- 05 253 171
- JP-A- 10 210 776
- JP-A- 58 044 033
- JP-A- 63 220 781
- JP-U- 02 083 695
- US-A- 5 079 471
- US-A1- 2005 052 094

## Description

### Technical Field

The present invention relates to an ultrasonic wave handling device including a stator with a working member hole and a columnar working member and a microscopic in-tube inspection system using the ultrasonic wave handling device, more specifically an ultrasonic wave handling device and a microscopic in-tube inspection system wherein the working member can freely revolve and/or slide (protrude and pull in).

### Background Art

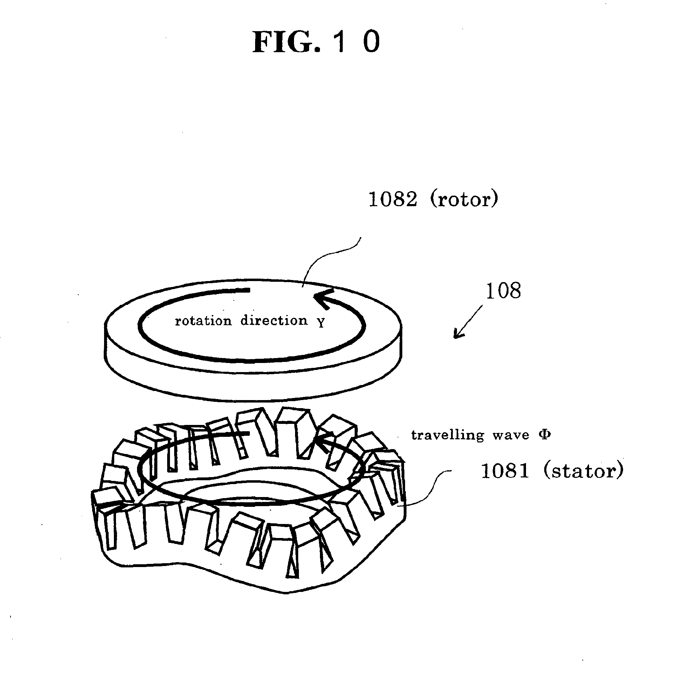

An ultrasonic wave motor is suitable to miniaturization because the structure is relatively simple and it has no coil. As shown in Fig. 10, a conventional ultrasonic wave motor 108 includes a ring shaped stator 1081 and a circular plate shaped rotor 1082, The stator 1081 and the rotor 1082 are pressure contacted and a cyclic vibration (a traveling wave ψ) is transmitted to the rotor 1082 from the surface of the stator 1081 and thereby the rotor 1082 rotates (rotation direction y).

US 2005/0052094 A1 describes different embodiments of an apparatus for driving a threaded shaft by activating piezoelectric plates designed to generate ultrasonic vibrations. Due to a multiplicity of threads on the surface of the threaded shaft, the generation of the ultrasonic vibrations results in a rotation of the threaded shaft, so that the threaded shaft rotating around its longitudinal axis is screwed along its longitudinal axis.

A similar ultrasonic wave handling device is also described in DE 44 35 996 A1 and US 5,079,471 A.

### Disclosure of Invention

However, the ultrasonic wave motor shown in Fig. 10 is not suitable for an application which requires a thin structure like a catheter because it needs a mechanism for pressure contacting (not shown) and has a large area circular shape in planner view.

Furthermore, because the area in planer view becomes larger (the diameter of the stator 1081 and the rotor 1082 becomes larger) in order to obtain a larger actuating force, the ultrasonic wave motor shown in Fig. 10 is not suitable for an application which requires a thin structure like a catheter.

On the other hand, a micro linear motor is used to slide an endoscope in an application like a catheter. The size of the liner motor portion becomes large because the micro linear motor needs to use a coil. The most popular catheter treatment is a blood clot treatment, and the blood clot is removed by putting dissolving drug on the blood clot from the tip portion of the catheter during this treatment. However the blood clot cannot be removed by dissolving dug if the blood clot is calcified or congealed, and therefore atherectomy catheters are developed.

An a herectomy catheter is a device for transmitting a torque to the revolving blade at the tip portion of the catheter through a torque transmission wire and crushing/removing a calcified or congealed blood clot by the revolving blade. However it is difficult to use the a herectomy catheter in a thin and complicated blood vessel like a cerebral blood vessel because the torque transmission is performed by a wire. It is also difficult to use in a heavily tortuous portion in the aorta.

If the tip portion of an atherectomy catheter can revolve without using a torque transmission wire, it can be sued in a thin and complicated cerebral blood vessel. Various techniques are developed to mount a microscopic ultrasonic wave handling device on the tip portion of a catheter, however the structure of a conventional device is complicated and no device with simple and flexible structure has been provided for usage in blood vessels.

It is known to apply a micro actuator with a revolving blade to an atherectomy catheter, however the size of a conventional micro actuator is large and such a micro actuator does not generate a practical torque.

A purpose of the present invention is to provide an ultrasonic wave handling device which resolves the aforementioned problems, more specifically an ultrasonic wave handling device which is flexible and allows revolution and axial movement (protruding and pulling in) of a working member and to provide a microscopic in-tube inspection system using the ultrasonic wave handling device.

The present invention provides the ultrasonic wave handling device with the features of claim 1.

An ultrasonic wave handling device according to the present invention can function as a revolving motor when a working member revolves and can function as a linear motor when the working member slides. An efficient revolving torque of the working member can be generated by making the outline of a cross section of the stator polygonal. In an ultrasonic wave handling device according to the present invention, it is preferable to drive the independently attached ultrasonic wave generating elements with 90°phase shift each other. This arrangement generates a travelling wave on the inner surface of the working member hole.

Since an ultrasonic wave handling device according to the present invention switches between revolution and sliding of the working member by controlling the drive frequency of the independently attached ultrasonic wave generating elements, it allows to control the drive frequency of the ultrasonic wave generating elements based on the resonant frequency in case of generating a revolution mode and the resonant frequency in case of generating a transition (sliding) mode, and therefore it is possible to appropriately switch between revolution and sliding of the working member.

In an ultrasonic wave handling device according to the present invention, an ultrasonic wave generating element can generate an ultrasonic wave in a first frequency band for revolving the working member and an ultrasonic wave in a second frequency band for (axially) sliding the working member.

In an ultrasonic wave handling device according to the present invention, it is also possible for an ultrasonic wave generating element to generate an ultrasonic wave generating element to generate an ultrasonic wave either in a first frequency band or in a second frequency band. In this case, one ultrasonic wave generating element is not used for generating an ultrasonic wave both in a first frequency band and in a second frequency band.

When the stators are arranged in a linear fashion, the working member can be rigid. When the stators are arranged in a curved fashion, it is possible to make the working member by a flexible material so that it can revolve in a bended condition or having a mechanism for allowing the working member to revolve in a bended condition. By this configuration, it is possible to operate in a microscopic tube as described below.

When an ultrasonic wave handling device according to the present invention has a working member which can slide (axially move with respect to the stator), the ultrasonic wave generating element which is attached to the stator can generate an ultrasonic wave in a first frequency band for revolving the working member and an ultrasonic wave in a second frequency band for axially moving the working member at a time or at different times.

In this case, the ultrasonic wave generating element generates an ultrasonic wave in a first frequency bend when it revolves the working member, generates an ultrasonic wave in a second frequency band when it slides (axially moves) the working member, and generates both an ultrasonic wave in a first frequency band and an ultrasonic wave in a second frequency band at a time when it axially moves the working member while it revolves the working member. That is to say, it is possible to use the ultrasonic wave generating element for generating an ultrasonic wave both in a first frequency band and in a second frequency band.

In an ultrasonic wave handling device according to the present invention, it is preferable to make the working member by a flexible material and cover the working member and one or more stators by a stretch tube except for the tip portion of the working member. In an ultrasonic wave handling device according to the present invention, since the ultrasonic wave handling device itself can be bended, it can be used as a catheter, for example.

In an ultrasonic wave handling device according to the present invention, an observation device and/or a processing device can be attached to the tip portion of the working member. A camera or an optical fiber collimator can be used as an observation device. A laser emitting device, a medical agent ejection device or a revolving blade can be used as a processing device.

A microscopic in-tube inspection system according to the present invention for using an ultrasonic wave handling device with an observation device and /or a processing device at the tip portion of the working member as a microscopic in-tube inspection robot, comprises the ultrasonic wave handling device, a power supply device for supplying energy to ultrasonic wave generating elements, and a control device for controlling the ultrasonic wave generating elements.

In a microscopic in-tube inspection system according to the present invention, an ultrasonic wave handling device can be configured as a microscopic in-tube inspection robot.

In an ultrasonic wave handling device according to the present invention, the working member can freely revolve and/or slide (axially moves). That is to say, an ultrasonic wave handling device according to the present invention can be used as a revolving motor, and in this case, a revolving functional member (a member exerting a functionality by revolution) like a blade, a mirror, etc., cam be attached to the tip portion of the working member which functions as a rotor.

An ultrasonic wave handling device according to the present invention can be used as a linear motor, and the working member can be formed in a shape which does not revolve (e.g. a prismatic column, an elliptic column) or can be configured in a revolution constrained structure. This structure can be used for focus control of an imaging device, a piston drive in a syringe (discharging of medical solution, or aspiration of blood).

### Brief Description of Drawings

Fig. 1 shows a schematic view of one embodiment of an ultrasonic wave handling device according to the present invention with a revolving working member, (A) shows an example where one ultrasonic wage generating element is attached to one side of the stator, and (B) shows an example where two ultrasonic wave generating elements are attached to one side of the stator.
Fig. 2 shows another embodiment of an ultrasonic wave handling device according to the present invention with a revolving working member, (A) shows an example where one ultrasonic wage generating element is attached to one side of the stator, and (B) shows an example where two ultrasonic wave generating elements are attached to one side of the stator.
Fig. 3 shows an experimental result for the relationship between the frequency of the applied voltage and the revolving speed/sliding speed.
Fig. 4 shows one embodiment of an ultrasonic wave handling device according to the present invention.
Fig. 5 shows an application of the present invention, an ultrasonic wave handling device for microscopic in-tube inspection used as a microscopic in-tube inspection robot.
Fig. 6 (A) shows one embodiment of a microscopic in-tube inspection system according to the present invention, and (B) shows the tip portion of the microscopic in-tube inspection system with an ultrasonic wave reflection mirror and an ultrasonic wave sensor mounted to the tip portion of the working member.
Fig. 7 shows an exemplary ultrasonic wave handling device with a tube-shaped working member.
Fig. 8 shows an example of an ultrasonic wave handling device with a stator having a slit, (A) shows an example where a slit is formed to enhance a displacement in a circumferential direction of the working member hole, and (B) shows an example where a slit is formed to enlarge the force in an axial direction of the working ember hole.
Fig. 9 shows a control mechanism of high-precision processing equipment formed by combining stators each other, working members each other, and a stator with a working member in an ultrasonic wave handling device according to the present invention.
Fig. 10 shows a schematic view of a conventional ultrasonic wave motor with a cylindrical stator.

### Best Mode for Carrying Out the Invention

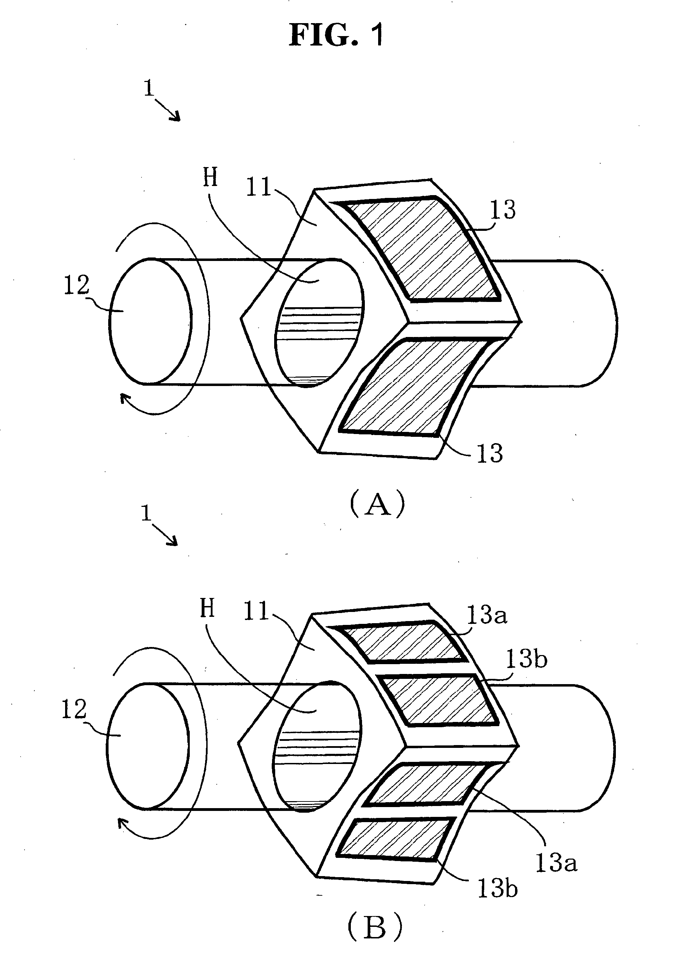

Fig. 1(A), (B) shows a schematic view of an ultrasonic wave handling device according to the present invention. In Fig. 1(A), an ultrasonic wave handling device 1 comprises a stator 11 having a working member hole H and a pillar shaped working member 12 which is inserted in the working member hole H. Ultrasonic wave generating elements (e.g. piezo elements) 13 for revolving the working member 12 are attached to the stator 11. In this ultrasonic wave handling device 1, the cross-sectional shape of the stator 11 on the plane which is perpendicular to the axis of the working member hole H is square and the stator 11 has four square sides encompassing the axis of the working member hole H. The ultrasonic wave generating elements 13 are attached to these square sides.

In Fig. 1(A), only two sides are shown, however the ultrasonic wave generating elements 13 are attached to four sides of the stator 11 respectively. These four ultrasonic wave generating elements 13 generate ultrasonic waves with 90° phase shift each other, and a travelling wave is generated on the inner surface of the working member hole H. As long as a travelling wave is generated on the inner surface of the working member hole H, the ultrasonic wave generating elements 13 can be attached to only two sides (adjacent two sides or opposite two sides), one side or three sides of the stator 11.

In the ultrasonic wave handling device 1, the stator 11 may have a diameter of 1 - 2mm and a length of 4 - 5mm, and thereby generates a maximum revolution speed of 1150 (rpm) and a maximum torque of several tens (µNm). The ultrasonic wave handling device 1 is suitable for medical applications, especially a catheter application.

In the ultrasonic wave handling device 1 shown in Fig. 1(A), the working member 12 revolves by the travelling wave generated in a circumferential direction on the inner surface of the working member hole H. The travelling wave is generated by the ultrasonic waves from the ultrasonic wave generating elements 13 on the four sides. As shown in Fig. 1(B), plural (two in Fig. 1(B)) ultrasonic wave generating elements 13a, 13b can be attached to one side so that these ultrasonic wave generating elements 13a, 13b generate in-phase or phase shifted ultrasonic waves.

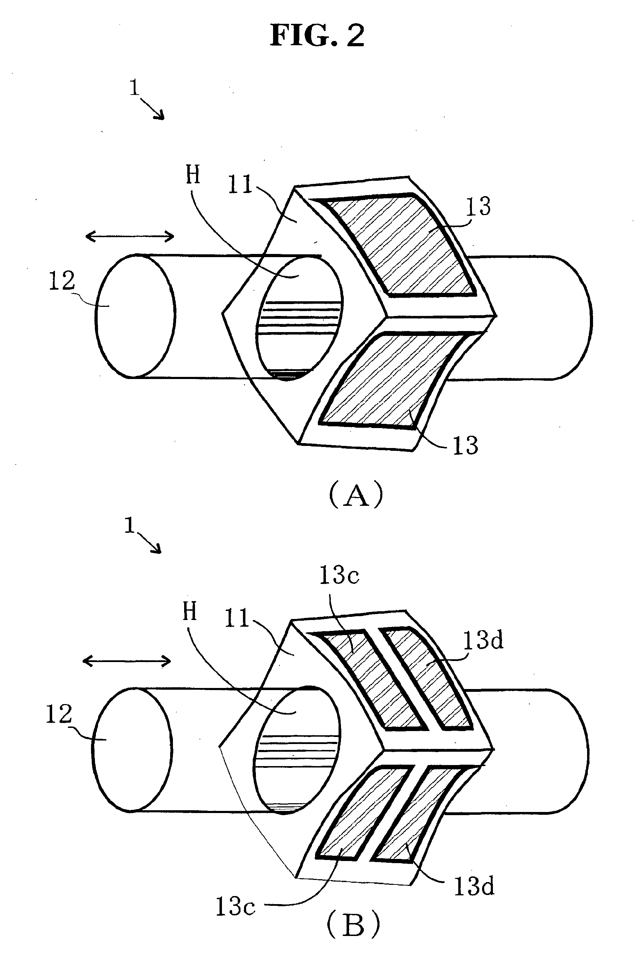

Fig. 2(A),(B) shows a schematic view of an ultrasonic wave handling device according to the present invention. In Fig. 2(A), (B), an ultrasonic wave handling device 1 comprises a stator 11 having a working member hole H and a pillar shaped working member 12 which is inserted in the working member hole H. Ultrasonic wave generating elements 13 for sliding the working member 12 are mounted on the stator 11.

The configuration of the ultrasonic wave handling device 1 shown in Fig. 2(A) is identical to that of the ultrasonic wave handling device 1 shown in Fig. 1(A). The configuration of the ultrasonic wave handling device 1 shown in Fig. 2(B) is identical to that of the ultrasonic wave handling device 1 shown in Fig. 2(A) except for the arrangement of the ultrasonic wave generating elements (e.g. piezo elements) 13.

In the ultrasonic wave handling device 1 shown in Fig. 2(A), the working member 12 slides by the travelling wave generated in an axial direction on the inner surface of the working member hole H. The travelling wave is generated by the ultrasonic waves from the ultrasonic wave generating elements 13 on the four sides. As shown in Fig. 2(B), plural (two in Fig. 2(B)) ultrasonic wave generating elements 13c, 13d can be attached to one side so that these ultrasonic wave generating elements 13c, 13d generate in-phase or phase shifted ultrasonic waves.

The working member hole H shown in Fig. 2(A),(B) has a circular cross section and the working member 12 has a circular pillar shape. However if the working member 12 slides only (does not revolve) in the working member hole H, the cross-sectional shape of the working member hole H is not limited to be circular and it can be ellipse, therefore the working member 12 does not need to have a circular pillar shape and can have other shape like an elliptic pillar shape.

Although the working member 12 revolves in the structure shown in Fig. 1(A),(B), it can slide by changing the frequency of the applied voltage. Although the working member 12 slides in the structure shown in Fig. 2(A),(B), it can also revolve by changing the frequency of the applied voltage.

An example for the devices shown in Fig. 1(A), (B) and Fig. 2(A), (B) was made as follows. The metal portion of the stator 11 had an area of 14mm x 14mm and a thickness of 10mm, and was made of the material C5191(phosphor bronze). The surface roughness of the faces where the ultrasonic wave generating elements were attached was Ra=1.6. The diameter of the working member hole H was 1.008mm. Piezo elements (Z0. 6T10x10S-W material C82) made by Fuji Ceramics Corp. was used as the ultrasonic wave generating elements. An epoxy resin curing agent was used as an adhesive agent. A stainless shaft which has a diameter of 9.998mm was used as the working member.

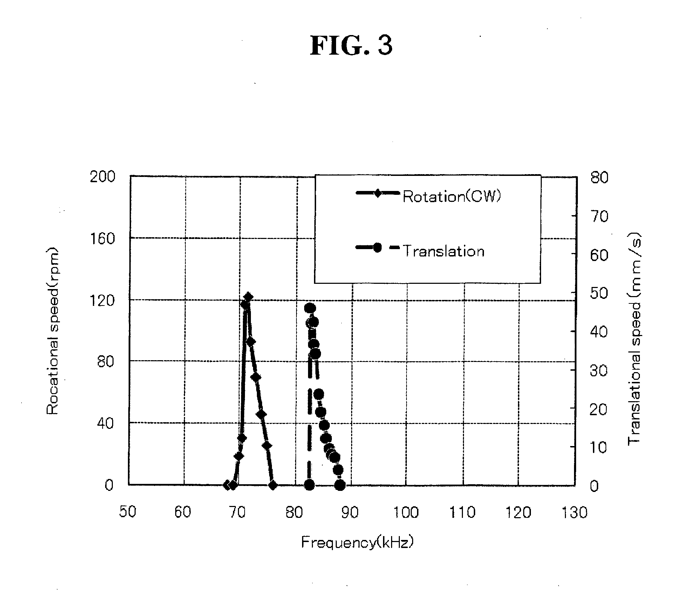

Fig. 3 shows an experimental result of the relationship between the frequency of the applied voltage and the revolving speed/sliding speed of the ultrasonic wave handling device 1. As shown by the solid line (rhombic plots) in Fig. 3, the ultrasonic wave handling device 1 shown in Fig. 1(A) operated at a revolving speed of 120rpm and with a torque of 2mNm when a voltage at a frequency of 71kHz was applied. As shown by the dashed line(dot plots) in Fig.3, the ultrasonic wave handling device 1 shown in Fig. 2(B) operated at a sliding speed of 50 (mm/s) and a drive force of 0.3N when a voltage at a frequency of 82kHz was applied.

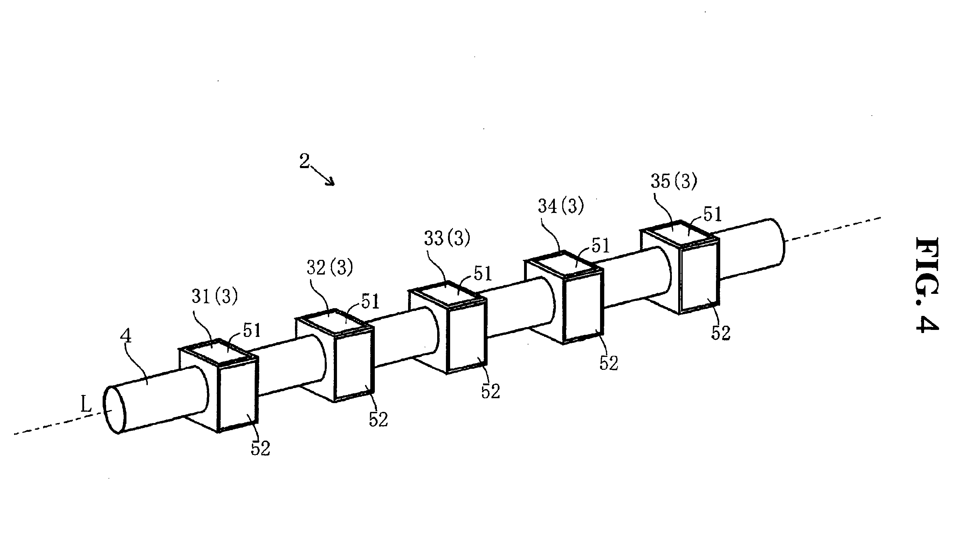

Fig. 4 shows a schematic view of one embodiment of an ultrasonic wave handling device according to the present invention. In Fig. 4, an ultrasonic wave handling device 2 is an inner circumference travelling wave type comprising a group of stators 3 and a working member 4. The group of stators 3 includes plural stators 31 through 35 each having a working member hole H and a working member 4 is fitted in each working member hole H of the stators 31 through 35. The number of the stators is five (reference numerals 31 through 35) in this embodiment, however the number of the stators can be two, three, four or six or more. The structure of the stators 31 through 36 may be identical to that of the stator 11 shown in Fig. 1(A),(B) and Fig. 2(A),(B). The outline of the cross section of the metal portion of the stators 31 through 35 which is perpendicular to the center axis L of the working member hole H forms a quadrate. The material of the metal is iron, stainless, aluminum, copper, etc. Four ultrasonic wave generating elements (51 through 54, 53 and 54 are not shown) are attached to four sides of the stators 31 through 35 respectively. The ultrasonic wave generating elements 51 through 54 in this embodiment are piezo elements as the cases shown in Figs. 1 and 2.

The ultrasonic wave generating elements 51 through 54 generate ultrasonic waves at first frequency band B1 for revolving the working member 4 and ultrasonic waves at second frequency band B2 for axially sliding the working member 4.

High frequency voltages having 90° phase shift each other are applied to the ultrasonic wave generating elements 51 through 54 from a power supply source(not shown). When the frequency of the high frequency voltage applied to the ultrasonic wave generating elements 51 through 54 is in first frequency band B1 (when the ultrasonic wave generating elements 51 through 54 generate ultrasonic waves in the first frequency band B1), the working member 4 revolves. When the frequency of the high frequency voltage applied to the ultrasonic wave generating elements 51 through 54 is in second frequency band B2 (when the ultrasonic wave generating elements 51 through 54 generate ultrasonic waves in the second frequency band B2), the working member 4 slides.

Although the working member 4 in the ultrasonic wave handling device 1 shown in Fig. 4 is configured to revolve and slide, it is also possible to configure the ultrasonic wave handling device to allow the working member to revolve only but does not allow the working member to slide or to allow the working member to slide only but does not allow the working member to revolve.

Since five stators 31 through 35 are used for one working member 4 in this embodiment, it is possible to make the drive force significantly large.

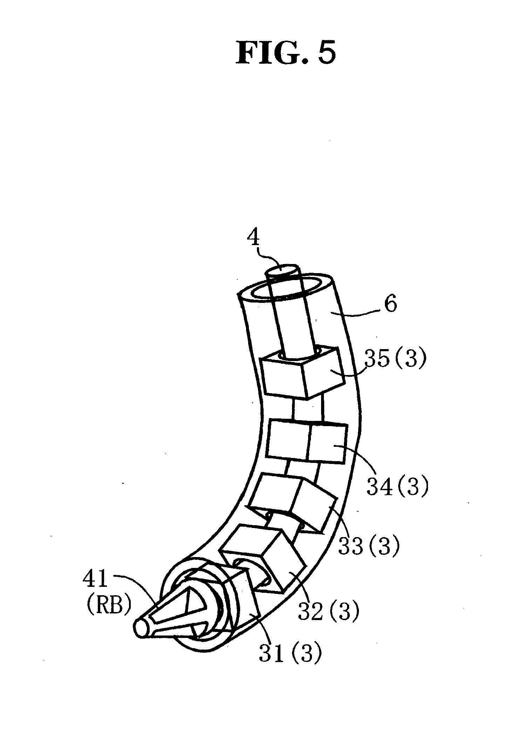

Fig. 5 shows a configuration of an ultrasonic wave handling device 1 for microscopic in-tube inspection purpose used as a microscopic in-tube inspection robot. In Fig. 5, the working member 4 is flexible and the stators 31 through 25 are arranged in a curved form (that is to say, the ultrasonic wave handling device itself can be curved). The working member 4 is configured to slide in an axial direction of the stators 31 through 35. A revolving blade 41 is attached to the top of the working member 4 as a functional member in Fig. 5. A blood clot removing cutter may be attached as a functional member in place of the revolving blade.

The working member 4 and the stators 31 through 35 are covered by a stretch tube (a silicon resin tube in this embodiment) 6 except for the tip portion of the working member 4. The stators 31 through 35 are arranged being bound by the stretch tube 6, and the revolving blade (RB) 41 at the tip portion of the working member 4 is exposed from the tip portion of the (flexible) stretch tube 6.

The flexible working member 4 is inserted in the working member hole H of the stators 31 through 35. The working member 4 can revolve by a revolving torque when the ultrasonic wave generating elements 51 through 54 of the stators 31 through 35 generate ultrasonic waves in the first frequency band B1. Since the stretch tube (silicon tube) 6 and the working member 4 are flexible, the ultrasonic wave handling device 1 is freely curved as a whole while it gives a revolving drive force to the working member 4. The working member 4 in the ultrasonic wave handling device 1 shown in Fig. 5 can slide (move in an axial direction) by applying a high frequency voltage in the second frequency band B2. That is to say, the revolving blade 41 at the tip portion of the working member 4 can be protruded and pulled in.

The ultrasonic wave handling device 1 shown in Fig. 5 can be miniaturized to the order of a diameter of 1 mm, and it is especially effective to use as a microscopic in-tube inspection robot for brain infarct treatment.

The drive frequencies can be different by making the shape of the stators 31 through 35 different each other in the ultrasonic wave handling device 1 shown in Fig. 5. The stators 31 through 35 may be independently controlled by using a common line (electric wire) as the connecting line for the ultrasonic wave generating elements of the stators 31 through 35 and changing the frequency of the applied voltage.

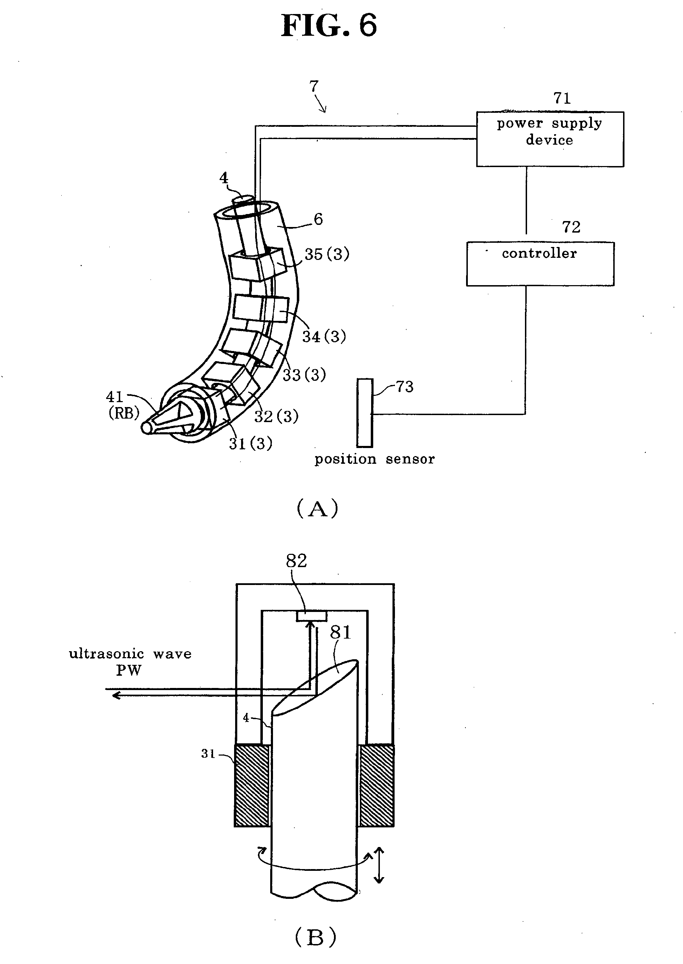

Fig. 6(A) shows a schematic view of one embodiment of a microscopic in-tube inspection system according to the present invention. The microscopic in-tube inspection system 7 shown in Fig. 6(A) comprises the ultrasonic wave handling device 1 (the microscopic in-tube inspection robot) shown in Fig. 5, a power supply device 71 for supplying energy to the ultrasonic wave generating elements, a controller 72 for controlling the ultrasonic wave generating elements (not shown in Fig. 6(A)) and a position sensor 73 for detecting the position of the ultrasonic wave handling device 1, and can be used with a MRI system at the same time.

Other functional members can be attached to the tip portion of the working member 4 in place of the revolving blade 41 in the microscopic in-tube inspection system 7. Fig. 6(B) shows one example of the microscopic in-tube inspection system 7 having an ultrasonic wave reflection mirror 81 and an ultrasonic wave sensor 82 at the tip portion of the working member 4 in place of the revolving blade 41. This microscopic in-tube inspection system 7 is used for Intravascular Ultrasound (IVUS) method, more specifically a method for revolving the ultrasonic wave reflection mirror 81 at the tip portion of the working member 4, scanning the inside of the blood vessel with an ultrasonic wave of 20-30 MHz generated by the ultrasonic wave sensor 82, and obtaining echo images for all 360° directions.

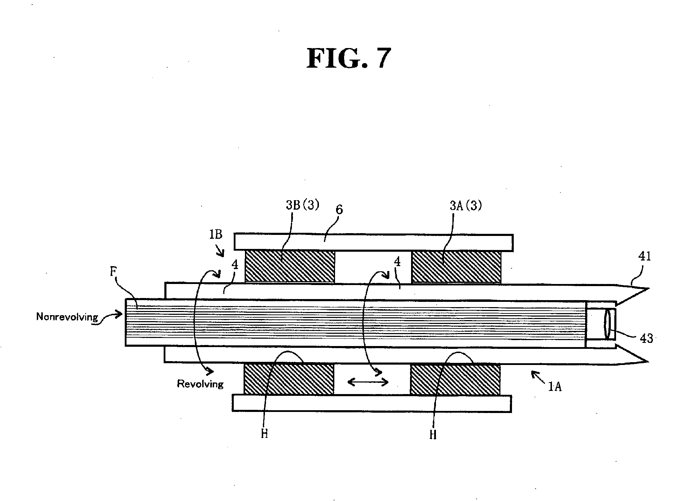

Fig. 7 shows one example of an ultrasonic wave handling device with a tube shaped working member 4. The ultrasonic wave handling device is covered by a rubber tube 6 as shown in Fig. 7. A revolving blade 41 is attached to the circumference of the tip of the working member (tube) 4, and an optical fiber F is contained in the inside of the tube.

An endoscope 43 is attached to the tip portion of the optical fiber F, and the working member 4 revolves in the stators 3A, 3B. At this time, the optical fiber F does not revolve. A medical doctor can remove the affected part by observing it through the optical fiber F.

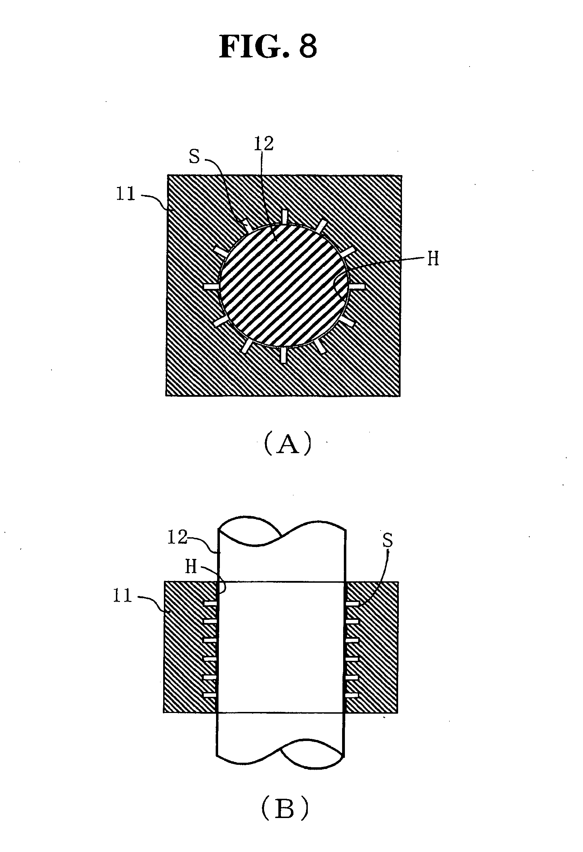

Fig. 8 (A), (B) shows one example of a stator 11 with a slit S in the ultrasonic wave handling device. The circumferential displacement is enhanced in the working member hole H according to the structure shown in Fig. 8 (A), and the axial force is enlarged in the working member hole H according to the structure shown in Fig. 8(B).

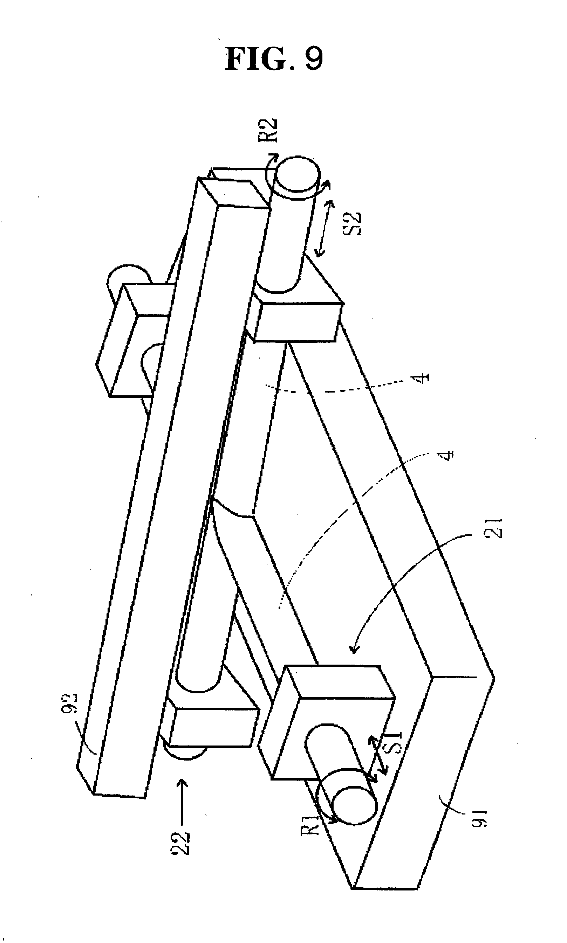

Fig. 9 shows a control mechanism for high-precision processing equipment formed by combining stators each other, working members each other, and a stator with a working member of the ultrasonic wave handling devices. In Fig. 9, a table includes a first support member and a second support member. The working member 4 of an ultrasonic wave handling device 21 attached to the first support member 91 is combined with the working member 4 of an ultrasonic wave handling devices 22 attached to the second support member 92.

Since the working member 4 of the ultrasonic wave handling device 21 can perform revolution R1 and sliding S1, and the working member 4 of the ultrasonic wave handling devices 22 can perform revolution R2 and sliding S2, a free attitude control of a stage can be realized by fixing the first support member 91 to a table and fixing the second support member 92 to a stage.

According to this embodiment, a table for high-precision processing equipment with a lot of flexibility by combining the stators each other, the working members each other, and the stator with the working member of the ultrasonic wave handling devices.

Although the preferable embodiments are explained by referring to the attached drawings, the scope of the present invention should not be interpreted to limit to the explained embodiments. A person skilled in the art can give thought to various alternative implementations and modified implementations within the scope of the claims and those implementations naturally fall within the scope of the present invention.

## Claims

1. Ultrasonic wave handling device (1 , 2, 7, 21 , 22) including one or more stators (3, 11) each having a hole (H) for accepting a working member (4, 12) and a pillar shaped working member (4, 12) which is inserted in the hole (H), wherein
ultrasonic wave generating elements (13, 51 , 52) for revolving and sliding the at least one working member (4, 12) are attached to the stators (3, 11),
the outline of the cross section of the stators (3, 11) which is perpendicular to the axial direction of the hole (H) forms a polygonal shape, and the ultrasonic wave generating elements (13, 51 , 52) are independently attached to each side of the polygonal stators (3, 11), and
the revolution and sliding of the working member (4, 12) is switched by controlling the drive frequency of the independently attached ultrasonic wave generating elements (13, 51, 52)
**characterized in that**
the ultrasonic wave generating elements (13, 51 , 52) generate ultrasonic waves of a first frequency band (B1) for revolving the at least one working member (4, 12) and ultrasonic waves of a second frequency band (B2) for axially sliding the at least one working member (4, 12).

2. Ultrasonic wave handling device (1, 2, 7, 21, 22) according to Claim 1, wherein
the working member (4, 12) is made of a flexible material, and the working member (4, 12) and one or more stators (3, 11) are covered by a stretch tube (6) except for the tip portion of the working member (4, 12).

3. Ultrasonic wave handling device (1, 2, 7, 21, 22) according to Claim 2, wherein
an observation device (82) and/or a processing device (81) are attached to the tip portion of the working member (4, 12).

4. Microscopic in-tube inspection system which uses an ultrasonic wave handling device (1, 2, 7, 21, 22) according to Claim 3 as a microscopic in-tube inspection robot, comprising the ultrasonic wave handling device (1, 2, 7, 21, 22), a power supply device (71) for supplying energy to the ultrasonic wave generating elements (13, 51, 52), and a control device (72) for controlling each of the ultrasonic wave generating elements (13, 51, 52).

## Patentansprüche

1. Ultraschallwellen-Verarbeitungsvorrichtung (1, 2, 7, 21, 22), die einen oder mehrere Statoren (3, 11) umfasst, die jeweils ein Loch (H) zum Aufnehmen eines Arbeitselements (4, 12) und ein säulenförmiges Arbeitselement (4, 12), das in das Loch (H) eingesetzt ist, aufweisen, wobei Ultraschallwellen-Erzeugungselemente (13, 51, 52) zum Drehen und Gleiten des mindestens einen Arbeitselements (4, 12) an den Statoren (3, 11) angebracht sind,
die Kontur des Querschnitts der Statoren (3, 11), der senkrecht zur axialen Richtung des Lochs (H) verläuft, eine polygonale Form bildet, und die Ultraschallwellen-Erzeugungselemente (13, 51, 52) unabhängig an jeder Seite der polygonalen Statoren (3, 11) angebracht sind, und
das Drehen und Gleiten des Arbeitselements (4, 12) durch Steuern der Ansteuerungsfrequenz der unabhängig angebrachten Ultraschallwellen-Erzeugungselemente (13, 51, 52) geschaltet wird,
**dadurch gekennzeichnet, dass**
die Ultraschallwellen-Erzeugungselemente (1-3, 51, 52) Ultraschallwellen eines ersten Frequenzbandes (B1) zum Drehen des mindestens einen Arbeitselements (4, 12) und Ultraschallwellen eines zweiten Frequenzbandes (B2) zum axialen Gleiten des mindestens einen Arbeitselements (4, 12) erzeugen.

2. Ultraschallwellen-Verarbeitungsvorrichtung (1, 2, 7, 21, 22) nach Anspruch 1, wobei das Arbeitselement (4, 12) aus einem flexiblen Material besteht und das Arbeitselement (4, 12) und ein oder mehrere Statoren (3, 11) durch einen Streckröhre (6), mit Ausnahme des Spitzenabschnitts des Arbeitselements (4, 12), bedeckt sind.

3. Ultraschallwellen-Verarbeitungsvorrichtung (1, 2, 7, 21, 22) nach Anspruch 2, wobei eine Beobachtungsvorrichtung (82) und/oder eine Verarbeitungsvorrichtung (81) an dem Spitzenabschnitt des Arbeitselements (4, 12) angebracht sind.

4. Mikroskopisches Röhreninspektionssystem, das mit einer Ultraschallwellen-Verarbeitungsvorrichtung (1, 2, 7, 21, 22) nach Anspruch 3 als einem mikroskopischen Röhreninspektionsroboter arbeitet, der Folgendes umfasst: die Ultraschallwellen-Verarbeitungsvorrichtung (1, 2, 7, 21, 22), eine Stromversorgungsvorrichtung (71) zum Zuführen von Energie zu den Ultraschallwellen-Erzeugungselementen (13, 51, 52) und eine Steuervorrichtung (72) zum Steuern eines jeden der Ultraschallwellen-Erzeugungselemente (13, 51, 52).

## Revendications

1. Dispositif de manipulation d'ondes ultrasonores (1, 2, 7, 21, 22) comprenant un ou plusieurs stators (3, 11) chacun possédant un trou (H) pour recevoir un élément de travail (4, 12) et un élément de travail en forme de colonne (4, 12) qui est inséré dans le trou (H), dans lequel des éléments de génération d'ondes ultrasonores (13, 51, 52) pour faire tourner et faire glisser ledit au moins un élément de travail (4, 12) sont fixés sur les stators (3, 11),
le contour de la section transversale des stators (3, 11) qui est perpendiculaire à la direction axiale du trou (H) forme une forme polygonale, et les éléments de génération d'ondes ultrasonores (13, 51, 52) sont fixées de façon indépendante sur chaque côté des stators polygonaux (3, 11), et
la rotation et le glissement de l'élément de travail (4, 12) sont activés en commandant la fréquence d'excitation des éléments de génération d'ondes ultrasonores fixés de façon indépendante (13, 51, 52)
**caractérisé en ce que**
les éléments de génération d'ondes ultrasonores (13, 51, 52) génèrent des ondes ultrasonores d'une première bande de fréquences (B1) pour faire tourner ledit au moins un élément de travail (4, 12), et des ondes ultrasonores d'une seconde bande de fréquences (B2) pour faire coulisser axialement ledit au moins un élément de travail (4, 12).

2. Dispositif de manipulation d'ondes ultrasonores (1, 2, 7, 21, 22) selon la revendication 1, dans lequel l'élément de travail (4, 12) est constitué d'un matériau souple, et l'élément de travail (4, 12) et un ou plusieurs stators (3, 11) sont recouverts par un tube extensible (6) à l'exception de la partie d'extrémité de l'élément de travail (4, 12).

3. Dispositif de manipulation d'ondes ultrasonores (1, 2, 7, 21, 22) selon la revendication 2, dans lequel un dispositif d'observation (82) et/ou un dispositif de traitement (81) sont fixés à la partie d'extrémité de l'élément de travail (4, 12).

4. Système d'inspection microscopique en tube qui utilise un dispositif de manipulation d'ondes ultrasonores (1, 2, 7, 21, 22) selon la revendication 3, tel qu'un robot d'inspection microscopique en tube, comprenant le dispositif de manipulation d'ondes ultrasonores (1, 2, 7, 21, 22), un dispositif d'alimentation électrique (71) pour fournir de l'énergie aux éléments de génération d'ondes ultrasonores (13, 51, 52), et un dispositif de commande (72) pour commander chacun des éléments de génération d'ondes ultrasonores (13, 51, 52).
